Europäisches Patentamt

European Patent Office

Office européen des brevets

(19)

(11) Veröffentlichungsnummer : **0 030 675**
**B1**

## (12) EUROPÄISCHE PATENTSCHRIFT

(45) Veröffentlichungstag der Patentschrift :
26.09.84

(51) Int. Cl.³ : **A 61 B   5/08**

(21) Anmeldenummer : **80107530.0**

(22) Anmeldetag : **02.12.80**

(54) **Auswechselbares Mundstück für Atemtestgeräte.**

(30) Priorität : **18.12.79 DE 2950970**

(43) Veröffentlichungstag der Anmeldung :
**24.06.81 Patentblatt 81/25**

(45) Bekanntmachung des Hinweises auf die Patenterteilung : **26.09.84 Patentblatt 84/39**

(84) Benannte Vertragsstaaten :
**DE FR GB IT NL**

(56) Entgegenhaltungen :
**DE-A- 2 125 998**
**FR-A- 1 348 260**
**GB-A- 2 064 324**
**US-A- 3 990 472**
**US-A- 4 139 469**

(73) Patentinhaber : **Siemens Aktiengesellschaft**
**Berlin und München Wittelsbacherplatz 2**
**D-8000 München 2 (DE)**

(72) Erfinder : **Ecker, Ernst, Dr. rer. nat.**
**Kirchhofstrasse 113**
**D-7500 Karlsruhe 31 (DE)**
Erfinder : **Schwelkert, Herbert**
**Wurttembergerstrasse 10**
**D-7514 Leopoldshafen (DE)**

### Beschreibung

Die Erfindung bezieht sich auf ein auswechselbares Mundstück für Atemtestgeräte mit einem zylindrischen Gehäuse, das mit einem Ende unverwechselbar auf den Gaseinlaßstutzen des Testgeräts aufsteckbar und am anderen Ende mit einer Öffnung zum Einblasen der Atemluft versehen ist, einem Rückschlagventil, dessen Ventilkörper in dem Gehäuse zwischen zwei Endlagen beweglich angeordnet ist, einem, die eine Endlage in Blasrichtung bestimmenden, gasdurchlässigen ersten Anschlag für den Ventilkörper und einem, die andere Endlage entgegen der Blasrichtung bestimmenden, als Ventilsitz für den Ventilkörper ausgebildeten zweiten Anschlag.

Derartige Mundstücke für den einmaligen Gebrauch sind beispielsweise aus der GB-A-20 64 324 bekannt. Das Rückschlagventil ist dort als eine Art Membranventil ausgebildet, der bewegliche Ventilkörper in Form einer flachen Membran oder Teilen davon wird im Atemstrom aus seiner Grundstellung herausgeschwenkt oder -gehoben und gibt so eine Durchtrittsöffnung frei, während er in Gegenrichtung dichtend an einem entsprechend ausgebildeten Ventilsitz anliegt.

Das bekannte Mundstück ist relativ einfach aufgebaut, ein Ansaugen von Luft aus dem Testgerät über des Mundstück wird wirksam verhindert.

Da im Atemstrom einer Testperson aber auch Speicheltröpfchen mitgeführt werden, die zu Beeinträchtigungen der Meßeingenschaften des Testgeräts führen, besteht die Forderung, in der Zuleitung zum Testgerät eine sogenannte Sputumfalle vorzusehen, um die Speicheltröpfchen möglichst weitgehend aus dem Gasstrom zu entfernen.

Es besteht die Aufgabe, eine solche Sputumfalle auf einfachste Weise zu schaffen und möglichst nahe der Testperson im Gasstrom anzuordnen.

Eine Lösung der Aufgabe wird in einem auswechselbaren Mundstück für Atemtestgeräte der eingangs genannten Art gesehen, bei welchem der Ventilkörper des Rückschlagventils eine Kugel aus saugfähigem, leichtem Material ist.

In einer bevorzugten Ausführungsform besteht die Kugel aus Zellstoff. Die als Ventilkörper bei Rückschlagventilen an sich bekannte Kugel (siehe z. B. US-A-39 90 472) dient hier zugleich als Tropfenfänger für die im Atemluftstrom mitgeführten Speicheltröpfchen, so daß sich eine besondere Ausbildung einer Sputumfalle erübrigt.

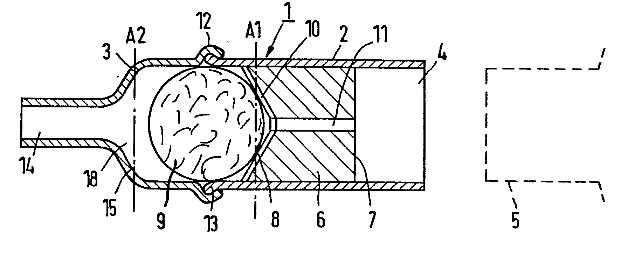

In der Figur ist ein Ausführungsbeispiel eines auswechselbaren Mundstücks gemäß der Erfindung im Längsschnitt dargestellt und im folgenden beschrieben.

Das im wesentlichen zylindrische Gehäuse 1 des Mundstücks besteht aus zwei koaxial zusammensteckbaren Teilen 2 und 3. Der zylindrische Teil 2 ist mit seinem einen Ende 4 auf den gestrichelt angedeuteten Gaseinlaßstutzen 5 des Atemtestgerätes aufsteckbar. In seinem Innern ist ein Formteil 6 aus einem vorzugsweise porösen Leichtwerkstoff so angeordnet, daß dessen dem Ende 54 zugewandte Stirnfläche 7 als Anschlag für den Rand des Gaseinlaßstutzens 5 wirkt und somit dessen Einstecktiefe festlegt. Die Stirnfläche 8 des Formteils 6 weist eine trichterförmige Vertiefung auf, die als erster hubbegrenzender Anschlag A1 für die als Ventilkörper eines Rückschlagventils in dem Gehäuse 1 beweglich angeordnete Kugel 9 in Blasrichtung dient. In der trichterförmigen Vertiefung der Stirnfläche 8 sind radial verlaufende Rinnen 10 angebracht, die in einen axialen Durchlaß 11 münden, zur Abführung der die Kugel 9 beim Einblasen umströmenden Atemluft.

Die Kugel 9 besteht aus einem sehr leichten und saugfähigen Material, beispielsweise aus Zellstoff, ihr Durchmesser ist so auf den Innendurchmesser des Gehäuses 1 abgestimmt, daß sich zwischen Kugeläquator und Gehäuseinnenwand ein schmaler Ringspalt bildet, durch den die Atemluft strömt. Der andere Teil 3 des Gehäuses 1 läßt sich mit seinem, mit einem rinnenförmigen Rand 12 versehenen Ende auf den Randwulst 13 des Gehäuseteils 2 aufschieben und dort federnd verrasten.

Der Gehäuseteil 3 weist entgegen der Blasrichtung eine Querschnittsverengung auf, die von dem Gehäusedurchmesser auf den kleineren Durchmesser der Einblasöffnung 14 überleitet. Die Übergangsschulter 15 zwischen den beiden Querschnitten des Gehäuseteils 3 ist so ausgebildet, daß sie als Ventilsitz 18 für die Kugel 9 und damit als hubbegrenzender zweiter Anschlag A2 in Gegenblasrichtung dient.

Zur Funktion : Zum Gebrauch wird das Mundstück, wie beschrieben, auf den Gaseinlaßstutzen 5 des Atemtestgerätes aufgesteckt, die Testperson bläst durch die Einblasöffnung 14 in das Testgerät. Beim Anblasen wird die aus sehr leichtem Material bestehende Kugel 9 in ihre eine Endlage, nämlich den ersten Anschlag A1 in der Stirnfläche 8, gedrückt, die Atemluft umströmt die Kugel und tritt durch den zwischen ihr und dem Gehäuse gebildeten Ringspalt, die radial verlaufenden Rinnen 10 und den Durchlaß 11 in den Gaseinlaßstutzen 5 ein. In der Atemluft mitgeführte Speicheltröpfchen werden in der als Sputumfalle wirkenden saugfähigen Kugel 9 festgehalten.

Wird die Strömungsrichtung durch Ansaugen der Luft aus dem Testgerät umgekehrt, bewegt sich die Kugel 9 infolge ihrer Leichtigkeit sehr schnell in ihre Endlage auf den Ventilsitz 18 und wirkt so als nahezu verzugsfrei ansprechendes Rückschlagventil, welches die Strömung in Gegenblasrichtung unterbricht.

### Ansprüche

1. Auswechselbares Mundstück für Atemtestgeräte mit

— einem zylindrischen Gehäuse (1), das mit einem Ende (4) unverwechselbar auf den Gaseinlaßstutzen (5) des Testgeräts aufsteckbar und am anderen Ende mit einer Einblasöffnung (14) versehen ist,

— einem Rückschlagventil, dessen Ventilkörper in dem Gehäuse (1) zwischen zwei Endlagen beweglich angeordnet ist,

— einem, die eine Endlage in Blasrichtung bestimmenden, gasdurchlässigen ersten Anschlag (A1) für den Ventilkörper,

— einem, die andere Endlage entgegen der Blasrichtung bestimmenden, als gasdichter Ventilsitz für den Ventilkörper ausgebildeten zweiten Anschlag (A2),
dadurch gekennzeichnet, daß der Ventilkörper des Rückschlagventils eine Kugel (9) aus saugfähigem, leichtem Material ist.

2. Mundstück nach Anspruch 1, dadurch gekennzeichnet, daß die Kugel (9) aus Zellstoff besteht.

## Claims

1. An exchangeable mouthpiece for breathing test apparatus comprising

— a cylindrical housing (1) which is non-exchangeably attachable on the gas inlet connecting piece (5) of the test apparatus by means of one end (4) and is provided at the other end with an opening (14) for blowing in,

— a non-return valve, the valve body of which is arranged in the housing (1) between two end positions,

— a first stop (A1) for the valve body which is permeable to gas and determines the one end position in the blowing direction,

— a second stop (A2) which is formed as a gastight valve seat for the valve body and which determines the other end position against the blowing direction,
characterised in that the valve body of the non-return valve is a ball (9) made of an absorbent, light material.

2. A mouthpiece according to Claim 1, characterised in that the ball (9) consists of cellulose.

## Revendications

1. Embout buccal interchangeable pour des appareils d'essai respiratoires comprenant :

— un boîtier cylindrique (1), dont l'une des extrémités (4) peut être emmanchée, d'une manière irréversible, sur le raccord d'entrée des gaz (5) de l'appareil d'essai et dont l'autre extrémité est munie d'une ouverture d'insufflation (14),

— un clapet anti-retour, dont le corps de clapet est disposé mobile dans le boîtier (1) entre deux positions d'extrémité,

— une première butée (A1) pour le corps de clapet, qui est perméable au gaz et qui détermine l'une des positions d'extrémité dans le sens d'insufflation,

— une seconde butée (A2) qui est agencée en siège étanche au gaz pour le corps de clapet et qui détermine l'autre position d'extrémité dans le sens opposé à celui d'insufflation,
caractérisé en ce que le corps de clapet du clapet anti-retour est une bille (9) en un matériau léger et absorbant.

2. Embout buccal suivant la renvendication 1, caractérisé en ce que la bille (9) est en cellulose.